# EUROPÄISCHE PATENTSCHRIFT

(11) **EP 3 831 291 B1**
(45) Veröffentlichungstag und Bekanntmachung des Hinweises auf die Patenterteilung: **19.02.2025**
(21) Anmeldenummer: 19213828.7
(22) Anmeldetag: 05.12.2019
(51) Int. Cl.: A61B 5/103, A61B 18/14

(54) **INSTRUMENT ZUR ELEKTROCHIRURGISCHEN BEHANDLUNG**
ELECTROSURGICAL TREATMENT INSTRUMENT
INSTRUMENT DE TRAITEMENT ÉLECTROCHIRURGICAL

(43) Veröffentlichungstag der Anmeldung: 09.06.2021
(73) Patentinhaber: Erbe Elektromedizin GmbH, 72072 Tübingen (DE)
(72) Erfinder: Keller, Sandra, 72379 Hechingen (DE); Enderle, Markus D., 72070 Tuebingen (DE); Ederer, Michael, 70563 Stuttgart (DE)
(74) Vertreter: Rüger Abel Patentanwälte PartGmbB

(56) Entgegenhaltungen:
- DE-A1- 102008 004 843
- US-A1- 2004 106 937
- US-A1- 2008 009 747
- US-A1- 2017 020 395
- US-B2- 9 155 587

## Beschreibung

Die Erfindung betrifft ein Instrument, insbesondere eine Sonde oder endoskopische Sonde, das zur thermischen Behandlung, beispielsweise elektrochirurgischen Behandlung von Gewebe eingerichtet ist. Das Instrument bzw. die Sonde kann mittels eines festen Körpers oder auch mittels eines Plasmas thermisch auf das Gewebe einwirken. Das Instrument kann beispielsweise zur Kauterisierung oder zur Plasma-Koagulation, insbesondere der Argonplasma-Koagulation eingerichtet sein.

Ein derartiges Instrument ist beispielsweise aus DE 198 20 240 A1 bekannt. Das Instrument kann durch einen Arbeitskanal eines Endoskops geführt werden. Im Anschluss an das distale Ende weist das Instrument Markierungsringe auf, mittels denen ein Operateur erkennen kann, wie weit das distale Ende aus dem Endoskop herausragt.

US 9 155 587 B2 beschreibt ein Instrument zur Behandlung von Gewebe. Die zu behandelnden Bereiche können intravaskulär mit dem Instrument erreicht werden, beispielsweise innerhalb des Herzens. Gewebebereiche können dabei abgetragen werden, beispielsweise mittels eines elektrolytischen Fluids.

Weitere Instrumente zum Abtragen oder Behandeln von Gewebe sind beispielsweise aus US 2004/0106937 A1, US 2017/0020395 A1, US 2008/0009747 A1 sowie DE 10 2008 004 843 A1 bekannt.

Bei der thermischen und insbesondere elektrochirurgischen Behandlung von Gewebe ist die Dosierung der Behandlung für den Operateur häufig schwierig abzuschätzen, also wie lange das Gewebe im Applikationsbereich thermisch behandelt werden muss bzw. welche Energie pro Flächeneinheit eingetragen werden muss, um die gewünschte Tiefenwirkung zu erreichen. Das Gewebe darf weder zu kurz, noch zu lang behandelt werden, um eine Unterdosierung bzw. Überdosierung zu vermeiden und den gewünschten Effekt, insbesondere die gewünschte Tiefenwirkung zu erzielen.

Ausgehend hiervon ist es eine Aufgabe der vorliegenden Erfindung eine Sonde zu schaffen, die das Dosieren, insbesondere das Abschätzen der Dauer der Behandlung im Applikationsbereich vereinfacht.

Diese Aufgabe wird durch eine Sonde mit den Merkmalen des Patentanspruches 1 gelöst.

Das Instrument bzw. die Sonde ist zur thermischen Behandlung von Gewebe eingerichtet. Das Gewebe wird in einem Applikationsbereich, insbesondere mittels eines Plasmas behandelt. Vorzugsweise ist das Instrument bzw. die Sonde zur elektrochirurgischen Koagulation eingerichtet, insbesondere zur Argonplasma-Koagulation.

Das Instrument weist einen Instrumentenkörper auf, der abhängig von der Ausführung des Instruments bzw. der Sonde durch einen bei den üblicherweise auftretenden Kräften nicht biegbaren, starren Rohrkörper oder durch einen flexiblen Schlauchkörper gebildet sein kann. Der Instrumentenkörper erstreckt sich von einem proximalen Ende zu einem distalen Ende. Am proximalen Ende kann der Instrumentenkörper an eine Versorgungs- und Bedieneinheit angeschlossen werden.

Das Instrument weist im Bereich des distalen Endes des Instrumentenkörpers eine Elektrode auf, an die eine Spannung, insbesondere eine hochfrequente Wechselspannung, angelegt werden kann. Die Elektrode kann über eine im Instrumentenkörper verlaufende elektrische Leitung mit einem elektrischen Anschluss verbunden sein, der insbesondere am proximalen Ende des Instruments bzw. der Sonde angeordnet ist. Eine Versorgungsleitung einer Versorgungs- uns Bedieneinheit kann mit dem elektrischen Anschluss verbunden werden, um die Spannung an die Elektrode anlegen zu können.

Mittels der Elektrode kann durch unmittelbaren Kontakt (z.B. bei der Kauterisierung) oder mittelbar über ein elektrisch leitfähiges Medium (z.B. bei der Koagulation) eine thermische Beeinflussung des Gewebes erfolgen.

Bei einem Ausführungsbeispiel ist das Instrument bzw. die Sonde zur Plasma-Koagulation, insbesondere der Argonplasma-Koagulation, eingerichtet. Dann kann in dem Instrumentenkörper ein Fluidkanal gebildet sein, der fluidisch mit einer Austrittsöffnung im Bereich des distalen Endes verbunden ist. Vorzugsweise ist die Elektrode im Bereich der Austrittsöffnung innerhalb des Instrumentenkörpers angeordnet. Die Elektrode wird von einem Fluid, insbesondere einem Inertgas, einem Gasgemisch, oder einem dem Inertgas beigefügten Zusatzstoff umspült.

Bei dieser Ausgestaltung des Instruments kann in einem bevorzugten Anwendungsfall das aus der Austrittsöffnung austretende Fluid, insbesondere das Inertgas, mittels der Elektrode ionisiert werden und in einen elektrisch leitfähigen Aggregatzustand gebracht werden, indem es beispielsgemäß in ein Plasma überführt wird. Das Fluid wird im Applikationsbereich auf das Gewebe gerichtet. Aufgrund des elektrisch leitfähigen Aggregatszustandes des ausströmenden ionisierten Fluids wird Energie auf das Gewebe übertragen. Zur Ionisierung des Fluids, insbesondere des Argongases wird an die Elektrode eine hochfrequente Wechselspannung angelegt, die dem Instrument über die Versorgungs- und Bedieneinheit bereitgestellt werden kann. Das Ionisieren des Inertgases erfolgt im Bereich unmittelbar vor der Austrittsöffnung.

In einem Anwendungsfall wird die über die Elektrode geleitete Energie in den Applikationsbereich auf das Gewebe übertragen, beispielsweise mittels Kontakt der Elektrode zum Gewebe. Die an der Elektrode übertragene Energie wird über eine hochfrequente Wechselspannung realisiert, die dem Instrument über die Versorgungs- und Bedieneinheit bereitgestellt werden kann.

Im Bereich des distalen Endes ist am Instrumentenkörper wenigstens eine Farbmarkierung angeordnet, die eine definierte Farbe bzw. einen definierten Farbton und/oder eine definierte Farbsättigung und/oder eine definierte Helligkeit hat, wobei die Farbe beispielsweise braun, braunbeige oder rot sein kann. Die wenigstens eine Farbmarkierung ist von außen sichtbar angeordnet und kann beispielsweise einen farbigen Aufdruck und/oder einen farbigen Einsatz aufweisen. Zusätzlich oder alternativ kann auch ein Bestandteil des Instrumentenkörpers in der gewählten oder definierten Farbe hergestellt und/oder zumindest teilweise gefärbt oder bedruckt sein, wie zum Beispiel ein die Austrittsöffnung aufweisendes Endstück. Die wenigstens eine Farbmarkierung kann vorzugsweise auf einer Mantelfläche des Instrumentenkörpers angebracht sein.

Die wenigstens eine Farbmarkierung kann wenigstens ein Symbol und/oder wenigstens ein Zeichen (Ziffer und/oder Buchstabe) und/oder wenigstens eine geometrische Figur, wie zum Beispiel ein Logo und/oder einen Barcode aufweisen. Beispielsweise kann wenigstens ein farbiger Ring als Farbmarkierung dienen. Die Farbmarkierung oder zumindest eine der Farbmarkierungen ist vorzugsweise durch eine zusammenhängende, vollständig gefüllte Fläche in der gewählten bzw. definierten Farbe gebildet.

Die Farbe der Farbmarkierung ist so gewählt, dass sie der Farbe eines behandelten Gewebes mit der gewünschten Dosierung und/oder Tiefenwirkung der Behandlung entspricht. Das heißt, die Farbmarkierung dient zum Farbvergleich mit der Färbung des behandelten Gewebes und die Färbung des behandelten Gewebes ist der Farbe der Farbmarkierung dann am ähnlichsten, wenn die gewünschte Dosierung bzw. Tiefenwirkung erreicht wurde. Ein Operateur kann daher unmittelbar durch Farbvergleich zwischen der Farbmarkierung und einem behandelten Gewebe erkennen, ob die Dosierung bzw. Einwirkungsdauer richtig gewählt wurde und somit einen Rückschluss auf die erzielte Tiefenwirkung ziehen. Für einen typischen Anwendungsfall gilt: ist die Farbe des behandelten Gewebes heller als die Farbe der Farbmarkierung, wurde die Behandlung nicht ausreichend lang durchgeführt. Ist die Farbe des behandelten Gewebes dunkler als die Farbe der Farbmarkierung, wurde die Behandlung überdosiert, insbesondere bei zu langer Behandlung des Gewebes. Sind die Farbe des behandelten Gewebes und der Farbmarkierung gleich, wurde die richtige Dosierung und Behandlungsdauer durchgeführt und die gewünschte Tiefenwirkung im Gewebe erreicht. Bei anderen Anwendungsfällen kann die Farbänderung auch abweichend sein vom beschriebenen typischen Anwendungsfall. Jedenfalls gibt die wenigstens eine Farbmarkierung die Farbe an, bei der für den betreffenden Anwendungsfall die gewünschte Dosierung bzw. Tiefenwirkung im Gewebe erreicht wurde.

Die Farbe der Farbmarkierung ist vorzugsweise braun oder braunbeige. Die Farbe, der Farbton oder ein anderes Farbmerkmal kann insbesondere abhängig von der Art der Behandlung und von dem zu behandelnden Gewebetyp ausgewählt werden. Bei einer Ausführungsform ist die Farbe der wenigstens einen Farbmarkierung eine RAL-Farbe. Je nach klinischer Indikation und Behandlungsgebiet bzw. Gewebetyp können für die wenigstens eine Farbmarkierung beispielsweise RAL-Farben mit den RAL-Nummern 1001, 1005, 1011, 1024, 8001, 8003, 8007 oder 8011 verwendet werden. Es hat sich gezeigt, dass diese RAL-Farben die Farbtöne des mit der richtigen Dosierung behandelten Gewebes sehr gut wiedergibt, wobei eine der RAL-Farben ausgewählt wird abhängig von der Art der Behandlung und von dem zu behandelnden Gewebetyp. Außerdem ist die RAL-Farbe genau definiert und lässt sich bei der Herstellung von Sonden sehr gut reproduzieren.

Um dem Operateur einen ausreichend genauen Vergleich zu gestatten, ist es vorteilhaft, wenn die Farbmarkierung ausreichend groß ist. Bevorzugt ist die Farbmarkierung flächig und weist bevorzugt eine Gesamtfläche von mindestens 15 mm² oder mindestens 20 mm² auf. Zusätzlich oder alternativ kann die Farbmarkierung in Erstreckungsrichtung des Instrumentenkörpers mindestens 2 mm oder mindestens 3 mm oder mindestens 5 mm lang sein. Es ist weiter bevorzugt, wenn die angegebene Gesamtfläche der Farbmarkierung als zusammenhängende Fläche ausgebildet und vorzugsweise vollständig mit der definierten Farbe ausgefüllt ist.

Bei einer bevorzugten Ausführungsform ist die eine Farbmarkierung oder ist wenigstens eine von mehreren Farbmarkierungen an einer Mantelfläche des Instrumentenkörpers vorhanden, insbesondere in Form eines Aufdrucks. Diese eine Farbmarkierung kann hier die Form von wenigstens einem Streifen oder Ring aufweisen.

Bei einer bevorzugten Ausführungsform ist die eine Farbmarkierung oder ist wenigstens eine von mehreren Farbmarkierung an einer Mantelfläche des Instrumentenkörpers vorhanden, insbesondere in Form einer zumindest teilweise umlaufenden Fläche, wie zum Beispiel eines umlaufenden Rings und/oder zwei oder mehreren Streifen. Es ist vorteilhaft, wenn sich diese wenigstens eine Farbmarkierung in einer Umfangsrichtung um den Instrumentenkörper erstreckt, zumindest um 50% des Umfangs des Instrumentenkörpers. Vorzugsweise erstreckt sich die wenigstens eine Farbmarkierung vollständig um den Umfang des Sondenkörpers herum und weist die Form einer geschlossenen Ringfläche auf.

Bevorzugt weist der Instrumentenkörper außerhalb der wenigstens einen Farbmarkierung eine Farbe oder mehrere Farben auf, die sich von der Farbe der wenigstens einen Farbmarkierung unterscheidet bzw. unterscheiden, und die sich insbesondere optisch vom Gewebe abhebt. Dadurch wird die Sichtbarkeit des Instruments bzw. der Sonde für den Operateur verbessert. Beispielsweise kann der Instrumentenkörper außerhalb bzw. umgebend zu der wenigstens einen Farbmarkierung blau sein. Die die wenigstens eine Farbmarkierung umgebende Farbe des Instrumentenkörpers ist bevorzugt dunkler als die Farbe der wenigstens einen Farbmarkierung.

Es ist bevorzugt, wenn die wenigstens eine Farbmarkierung nicht bis zum distalen Ende des Instrumentenkörpers reicht, sondern mit Abstand dazu angeordnet ist.

Es ist außerdem bevorzugt, wenn die wenigstens eine Farbmarkierung eine Markierung darstellt, die einen definierten Abstand oder Mindestabstand zum distalen Ende angibt. Dadurch kann dem Operateur angezeigt werden, wann der Instrumentenkörper bzw. das distale Ende weit genug aus einem Endoskop herausgeschoben wurde, um beim Applizieren des Plasmas die Optik des Endoskops nicht zu beschädigen oder zu beeinträchtigen. Beispielsweise kann der am weitesten entfernte Rand der wenigstens einen Farbmarkierung einen Mindestabstand zum distalen Ende angeben. Erst wenn die wenigstens eine Farbmarkierung vollständig durch eine Optik eines Endoskops zu erkennen ist, befindet sich das distale Ende des Instrumentenkörpers weit genug vom Endoskop weg, um eine Behandlung auszuführen.

Bei einem Ausführungsbeispiel weist der Instrumentenkörper ein Endstück auf, das die Austrittsöffnung aufweist. Das Endstück kann beispielsweise dazu eingerichtet sein, die Ausströmrichtung des Fluids bzw. des Plasmas vorzugeben. Das Endstück kann vollständig oder teilweise in der definierten Farbe der Farbmarkierung ausgeführt sein, so dass das Endstück zumindest eine der Farbmarkierungen bildet.

Die wenigstens eine Farbmarkierung oder wenigstens eine von mehreren Farbmarkierungen kann auch eine Textur und/oder Oberflächenstruktur und/oder mattierte und/oder nicht spiegelnde Oberfläche aufweisen. Dadurch kann die reale Gewebestruktur besser abgebildet und der Vergleich zwischen der texturierten Farbmarkierung und der Farbe und/oder Struktur des Gewebes vereinfacht werden. Außerdem kann eine raue und/oder matte und/oder nicht spiegelnde Oberfläche der Farbmarkierung Lichtspiegelungen verhindern. Die Oberfläche kann dabei vorzugsweise eine Rauheit aufweisen, die einer der VDI-Klassen 33 - 42 der VDI-Richtlinie 3400 (Oberflächenvergleichsmustermessung) entspricht. Die Oberfläche kann eine maximalen Rautiefe Rmax = 18 - 49 µm, insbesondere Rmax = 25 µm aufweisen und/oder einen arithmetischen Mittenrauwert Ra = 4,5 - 12,5 µm, insbesondere Ra = 6,3 µm aufweisen.

Die vorstehend erläuterten Ausgestaltungen der wenigstens einen Farbmarkierung können beliebig miteinander kombiniert werden. Beispielsweise kann sowohl ein Endstück des Instrumentenkörpers als eine Farbmarkierung dienen, als auch zusätzlich an der Mantelfläche des Instrumentenkörpers eine oder mehrere weitere farbige Flächen, Symbole, Zeichen oder dergleichen vorhanden sein. Bei einer bevorzugten Ausführungsform ist die wenigstens eine Farbmarkierung ausschließlich außen auf der Mantelfläche des Instrumentenkörpers vorhanden. In diesem Fall können die Instrumentenkörper unabhängig von der Art und Ausgestaltung der Austrittsöffnung bei der Herstellung von Sonden als Gleichteil verwendet werden. Dadurch lassen sich Skalierungseffekte erreichen.

Bei einem Ausführungsbeispiel ist der Instrumentenkörper als Instrumentenschlauch ausgebildet. Der Instrumentenschlauch ist quer zu seiner Erstreckungsrichtung bei den auftretenden Kräften flexibel bzw. biegbar. Vorzugsweise ist der Instrumentenschlauch dazu eingerichtet, durch einen Arbeitskanal eines Endoskops geführt zu werden.

Vorteilhafte Ausgestaltungen der Erfindung ergeben sich aus den abhängigen Ansprüchen, der Beschreibung und den Zeichnungen. Nachfolgend werden bevorzugte Ausführungsbeispiele der Erfindung anhand der beigefügten Zeichnungen im Einzelnen erläutert. Es zeigen:
Figur 1 eine schematische Prinzipdarstellung eines Endoskops sowie eines als Sonde ausgebildeten Instruments, die durch den Arbeitskanal des Endoskops geführt ist,
Figur 2 einen Endabschnitt der Sonde aus Figur 1, der aus dem Arbeitskanal des Endoskops herausragt in einer schematischen, blockschaltbildähnlichen Darstellung,
Figuren 3-5 und jeweils ein Ausführungsbeispiel unterschiedlich ausgestalteter Austrittsöffnungen einer Sonde für ein Plasma bzw. ein Fluid in einer schematischen Seitenansicht,
Figur 6a eine schematische Darstellung eines abgewandelten Ausführungsbeispiels des Endabschnitts einer Sonde,
Figur 6b eine schematische Darstellung eines abgewandelten Ausführungsbeispiels des Endabschnitts mit einer Farbmarkierung, die sich entlang des Endabschnittes einer Sonde erstreckt,
Figuren 7-9 schematische Prinzipdarstellungen einer Farbmarkierung einer Sonde im Vergleich zur Farbe eines Applikationsbereichs eines behandelten Gewebes,
Figur 10 eine photographische Darstellung eines Endabschnitts einer Sonde mit einer Farbmarkierung und unter mehreren Applikationsbereichen an einem Gewebe mit unterschiedlichen Anwendungsdosierungen der Sonde und
Figuren 11 und 12 jeweils weitere Ausführungsbeispiele einer Sonde in einer schematischen Teildarstellung.

Die Erfindung betrifft ein Instrument zur thermischen uns beispielsweise zur elektrochirurgischen Behandlung von biologischem Gewebe 34. Bei den nachfolgend anhand der Zeichnungen veranschaulichen Ausführungsformen ist das Instrument als Sonde 17 ausgebildet, die vorzugsweise zur Verwendung mit einem Endoskop 15 eingerichtet ist.

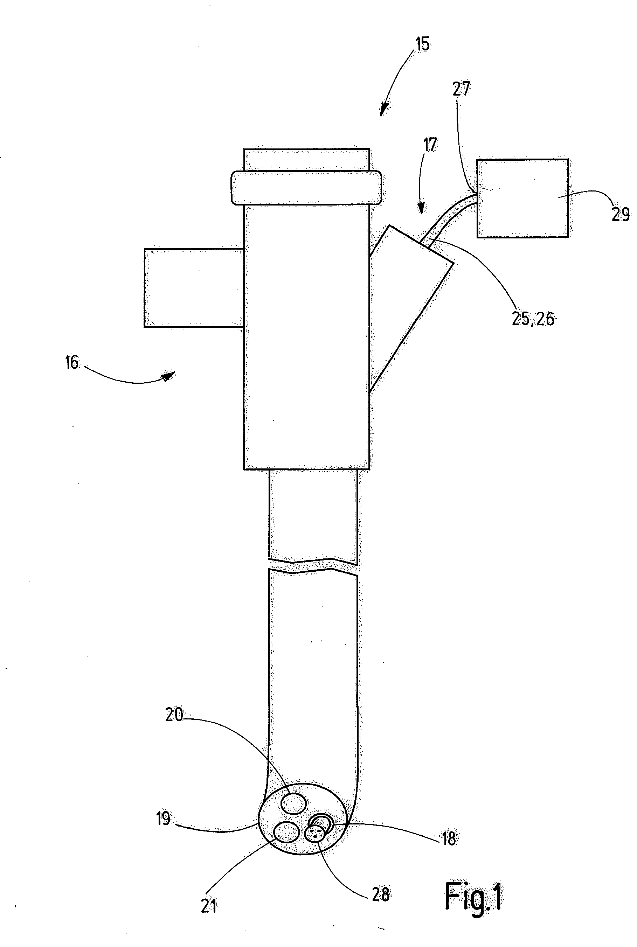

In Figur 1 ist das Endoskop 15 nach Art eines Blockschaltbilds veranschaulicht. Das Endoskop 15 weist einen vom Operateur handhabbaren Bedienteil 16 auf. Am Bedienteil 16 kann die Sonde 17 in einen Arbeitskanal 18 des Endoskops 15 eingeführt werden. Der Arbeitskanal 18 hat an einem dem Bedienteil 16 entgegengesetzten Endoskopende 19 eine Mündung, aus der zumindest ein Abschnitt der Sonde 17 herausgeführt werden kann. Benachbart zu der Mündung des Arbeitskanals 18 sind am distalen Endoskopende 19 eine Lichtquelle 20 und ein Objektiv 21 angeordnet. Über die Lichtquelle 20 wird die Umgebung vor dem Endoskopende 19 und insbesondere das zu behandelnde Gewebe beleuchtet. Mittels des Objektivs 21 und einer Optik oder Kamera wird ein Bild der Umgebung vor dem Endoskopende 19 und insbesondere des zu behandelnden Gewebes erfasst. Die Kamera und/oder Optik des Endoskops 15 leitet die Bilder an ein Okular und/oder einen externen Monitor weiter.

Das als Sonde 17 ausgeführte Instrument hat einen Instrumentenkörper 25, der beim Ausführungsbeispiel als quer zu seiner Erstreckungsrichtung bei den auftretenden Kräften biegbarer Instrumentenschlauch 26 ausgebildet ist. Da es sich bei dem Instrument um eine Sonde 17 handelt, könnte der Instrumentenkörper 25 auch als Sondenkörper und der Instrumentenschlauch als Sondenschlauch bezeichnet werden. Der Instrumentenkörper 25 und beispielsgemäß der Instrumentenschlauch 26 erstreckt sich von einem proximalen Ende 27 bis zu einem distalen Ende 28. An dem proximalen Ende 27 ist der Instrumentenschlauch 26 an eine Versorgungs- und Bedieneinheit 29 angeschlossen.

Das Instrument und beispielsgemäß die Sonde 17 ist bei den veranschaulichten Ausführungsformen zur Plasma-Koagulation, beispielsgemäß zur Argonplasma-Koagulation eingerichtet. Alternativ dazu könnte das Instrument auch zur Kauterisierung oder zur Durchführung einer anderen thermischen Behandlung von biologischem Gewebe eingerichtet sein.

Die Sonde 17 weist im Bereich des distalen Endes 28 des Instrumentenkörpers 25 eine Elektrode 32 auf. Die Elektrode 32 kann beispielsweise dazu eingerichtet sein, unmittelbar in Kontakt mit dem zu behandelnden biologischen Gewebe 34 gebracht zu werden. Beim bevorzugten Ausführungsbeispiel ist die Elektrode 32 dazu eingerichtet, mittelbar über ein elektrisch leitfähiges Medium eine thermische Beeinflussung des biologischen Gewebes 34 durchzuführen.

An die Elektrode 32 kann eine Spannung, insbesondere eine hochfrequente Wechselspannung, angelegt werden. Die Elektrode 32 ist über eine im Instrumentenkörper 25 verlaufende, nicht dargestellte elektrische Leitung mit einem elektrischen Anschluss verbunden, der insbesondere am proximalen Ende des Instruments bzw. der Sonde angeordnet ist. Über den elektrischen Anschluss kann eine elektrische Verbindung zur Versorgungs- uns Bedieneinheit 29 hergestellt werden, um die Spannung an die Elektrode 32 anlegen zu können.

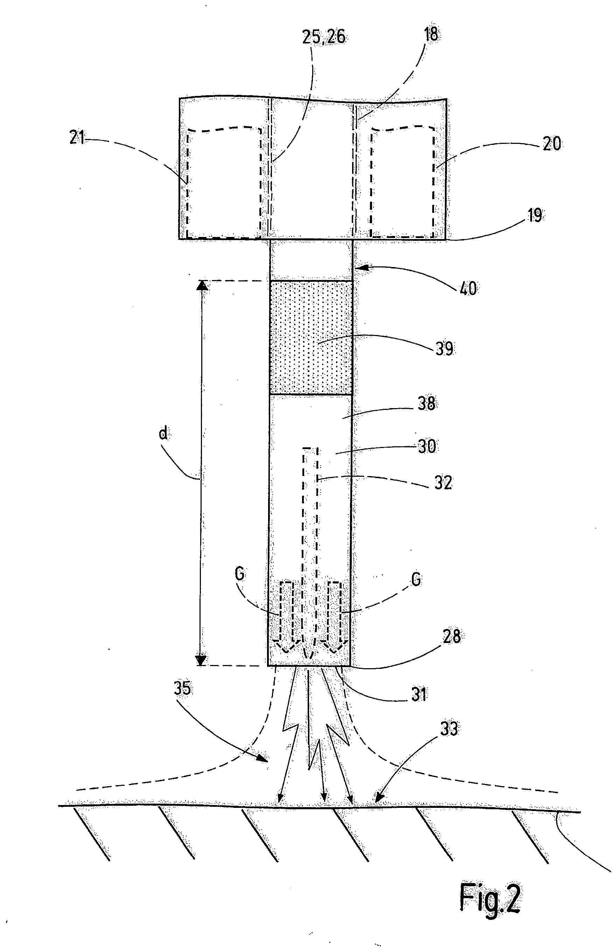

Bei der Ausgestaltung der Sonde 17 zur Plasma-Koagulation ist innerhalb des Instrumentenkörpers 25 bzw. des Instrumentenschlauchs 26 ist ein Fluidkanal 30 gebildet (Figur 2). Mittels der Versorgungs- und Bedieneinheit 29 kann ein Fluid G, wie zum Beispiel ein Inertgas, beispielsgemäß Argon, eingeleitet werden. Das Fluid G strömt durch den Fluidkanal 30 bis zu einer Austrittsöffnung 31 im Bereich des distalen Endes 28 des Instrumentenschlauchs 26.

Innerhalb des Instrumentenschlauchs 26 und benachbart zur Austrittsöffnung 31 ist die Elektrode 32 angeordnet. Wie erläutert ist die Elektrode 32 elektrisch mit der Versorgungs- und Bedieneinheit 29 verbindbar, so dass eine hochfrequente Wechselspannung an die Elektrode 32 angelegt werden kann. Mittels der hochfrequenten Wechselspannung kann das Fluid bspw. ein Inertgas G ionisiert und ein Plasma 35 gebildet werden. Wie es stark schematisiert in Figur 2 veranschaulicht ist, wird mittels des Plasmas und beispielsgemäß des Argonplasmas Energie in Richtung E des anliegenden elektrischen Feldes innerhalb eines Applikationsbereichs 33 auf ein zu behandelndes Gewebe 34 übertragen. Abhängig von der Dosierung, beispielsweise vom Betrag und/oder dem zeitlichen Verlauf der hochfrequenten Wechselspannung und/oder der Einwirkungsdauer auf den Applikationsbereich 33 wird durch den Energieeintrag mittels des Plasmas eine Tiefenwirkung im zu behandelnden Gewebe 34 erreicht. Die Dosierung des Energieeintrags, insbesondere der Einwirkungs- bzw. Behandlungsdauer in das zu behandelnde Gewebe 34 ist insbesondere dann optimal, wenn während der Behandlung etwa zwei Drittel der gewünschten Tiefenwirkung erreicht wird. Aufgrund von Nachwirkungseffekten innerhalb von etwa 72 Stunden nach der Behandlung nimmt die Tiefenwirkung um das noch fehlende Drittel zu.

Mittels der Sonde 17 kann somit eine thermische bzw. elektrochirurgische Behandlung und beispielsgemäß eine Argonplasma-Koagulation eines zu behandelnden Gewebes 34 ausgeführt werden. Die Argonplasma-Koagulation ist kontaktlos. Die Sonde 17 berührt das Gewebe 34 im Applikationsbereich 33 somit nicht.

In einem Endabschnitt 38 des Instrumentenkörpers 25 und beispielsgemäß des Instrumentenschlauchs 26 im Anschluss an sein distales Ende 28 ist wenigstens eine Farbmarkierung 39 vorhanden, die eine Farbe mit einem definierten Farbton und/oder einer definierten Farbsättigung und/oder einer definierten Helligkeit aufweist. Der Farbton kann beispielsweise ein Braun-Farbton sein. Die Farbe der Farbmarkierung 39 entspricht der Farbe, die das behandelte Gewebe 34 im Applikationsbereich 33 annimmt, wenn die durch die gewählte Dosierung eingetragene Energie zu der gewünschten Tiefenwirkung im Gewebe 34 führt. Die Farbe des behandelten Gewebes 34 im Applikationsbereich 33 ist daher ein Indikator für die erreichte Tiefenwirkung und die Dosierung, insbesondere die pro Flächeneinheit eingetragene Energie. Anhand der Farbmarkierung 39 kann ein optischer Vergleich zwischen der Farbe der Farbmarkierung und der Farbe des Gewebes 34 im Applikationsbereich 33 nach der Argonplasma-Koagulation durchgeführt werden. Es ist dann erkennbar, ob die Dosierung korrekt gewählt wurde.

Beispielsgemäß ist die Farbe der Farbmarkierung 39 braun oder braunbeige. Beim Ausführungsbeispiel ist die Farbe der wenigstens einen Farbmarkierung 39 die RAL-Farbe mit der Nummer 1011.

Die eine Farbmarkierung 39 oder wenigstens eine von mehreren Farbmarkierungen 39 weist eine oder mehrere zusammenhängende Farbflächen auf. Eine einzige zusammenhängende Farbfläche ist ausreichend.

Die eine Farbmarkierung 39 oder wenigstens eine von mehreren Farbmarkierungen 39 kann optional auch eine Textur und/oder Oberflächenstruktur und/oder mattierte Oberfläche aufweisen. Dadurch kann die reale Gewebestruktur besser abgebildet und der Vergleich zwischen der texturierten Farbmarkierung 39 und der Farbe und/oder Struktur des Gewebes 34 vereinfacht werden. Außerdem kann eine raue und/oder matte Oberfläche der Farbmarkierung 39 Spiegelungen verhindern, wodurch der Farbvergleich vereinfacht werden kann.

Sofern die eine Farbmarkierung 39 oder wenigstens eine von mehreren Farbmarkierung 39 eine oder mehrere Texturen aufweist, so können diese in Anlehnung an die VDI 3400 (Oberflächenvergleichsmustermessung) die VDI-Klassen 33 - 42 mit der maximalen Rautiefe Rmax = 18 - 49 µm und dem arithmetischen Mittenrauwert Ra = 4,5 - 12,5 µm, insbesondere die VDI-Klasse 36, Rmax = 25 µm, Ra = 6,3 µm ausgeführt werden.

Beim Ausführungsbeispiel ist zumindest eine Farbmarkierung 39 im Endabschnitt 38 auf eine Mantelfläche 40 bzw. Umfangsfläche des Instrumentenschlauchs 26 aufgebracht, beispielsweise durch ein Druckverfahren. Die Farbmarkierung 39 kann zum Beispiel eine die Mantelfläche 40 teilweise und vorzugsweise vollständig umschließende Ringfläche darstellen. In Erstreckungsrichtung des Instrumentenschlauchs 26 bzw. des Endabschnitts 38 weist die wenigstens eine Farbmarkierung 39 eine Länge von mindestens 2 mm oder mindestens 3 mm auf. Vorzugsweise ist die Länge der Farbmarkierung 39 in Erstreckungsrichtung des Endabschnitts 38 nicht größer als 5 mm oder 6 mm oder 7 mm oder 10 mm.

An dem Instrumentenschlauch 26 und insbesondere an der Mantelfläche 40 können weitere Markierungen für einen Operateur vorhanden sein, die in beliebigen Farben und Formen ausgestaltet werden können.

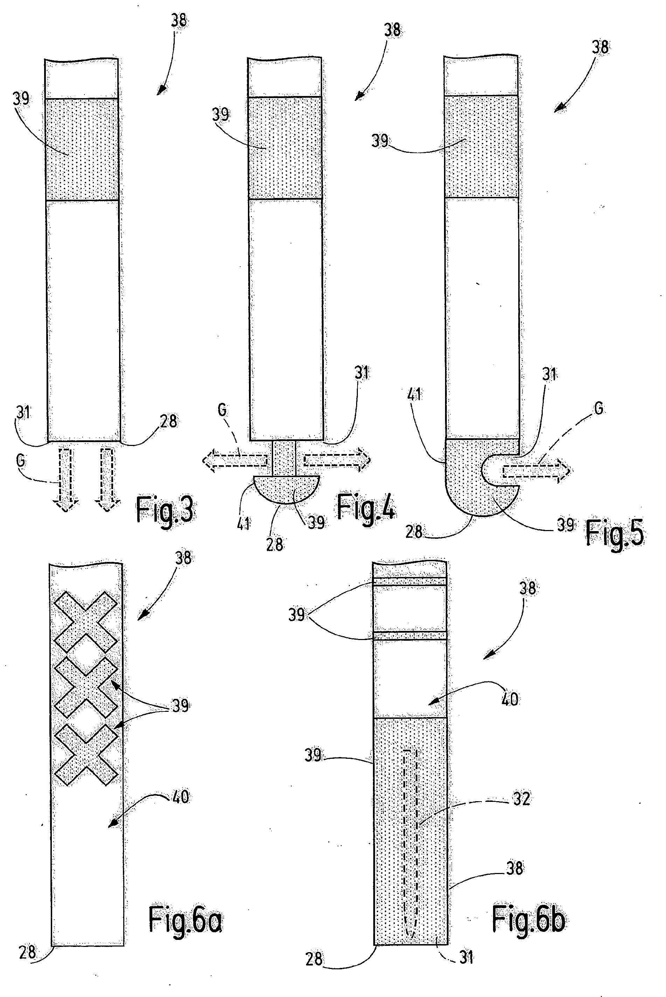

Anstelle einer Ringfläche kann die wenigstens eine Farbmarkierung 39 auch beliebige andere Ausgestaltungen aufweisen. Die wenigstens eine Farbmarkierung 39 kann beispielsweise wenigstens ein Symbol und/oder wenigstens ein Zeichen (Ziffer oder Buchstabe) und/oder wenigstens eine geometrische Figur aufweisen. Somit können auch ein Firmenschriftzug und/oder ein Firmenlogo als Farbmarkierung 39 verwendet werden. In Figur 6a ist schematisch der Aufdruck einer Farbmarkierung 39 in Form von Symbolen veranschaulicht.

Bei dem in Figur 6b veranschaulichten Endabschnitt 28 eines weiteren Ausführungsbeispiels der Sonde 17 ist zumindest eine der Farbmarkierungen 39 unmittelbar im Anschluss an das distale Ende 28 an der Mantelfläche 40 angebracht, beispielsweise aufgedruckt. Diese Farbmarkierung kann ganz oder teilweise durch ein hohlzylindrisches Endstück 41 gebildet sein. Eine oder mehrere weitere ringförmig Farbmarkierungen, die beispielsgemäß mit unterschiedlichen Abständen zum distalen Ende 28 angeordnet sind, können optional vorhanden sein.

Beim Ausführungsbeispiel der Sonde 17 gemäß der Figuren 2, 3 und 6a ist die eine Farbmarkierung 39 oder sind alle Farbmarkierungen 39 mit Abstand zum distalen Ende 28 des Instrumentenschlauchs 26 angeordnet. Bevorzugt hat der Instrumentenschlauch 26 abgesehen von der wenigstens einen Farbmarkierung 39 eine Farbe, die sich optisch vom umgebenden Gewebe abhebt, so dass der Instrumentenschlauch 26 während der Operation und beispielsgemäß des endoskopischen Eingriffs gut zu erkennen ist. Durch den Abstand der wenigstens einen Farbmarkierung 39 vom distalen Ende 28 kann die Austrittsöffnung 31 im erforderlichen Abstand zum Applikationsbereich 33 des Gewebes 34 positioniert werden.

Mittels der einen Farbmarkierung 39 oder - sofern mehrere Farbmarkierungen 39 vorhanden sind - einer der vorhandenen Farbmarkierungen 39 kann ein Mindestabstand d vom distalen Ende 28 des Instrumentenschlauchs 26 markiert werden. Beispielsweise kann ein Rand der Farbmarkierung 39, insbesondere der dem distalen Ende 28 entgegengesetzte Rand der Farbmarkierung 39 an einer Stelle des Endabschnitts 38 des Instrumentenschlauchs 26 angeordnet sein, der dem Mindestabstand d zum distalen Ende 28 angibt. Beim Endoskopieren kann der Operateur erkennen, ob die Sonde 17 bzw. deren Endabschnitt 38 weit genug aus dem Arbeitskanal 18 des Endoskops 15 herausgeschoben wurde, um während der Behandlung des Gewebes 34 das Endoskop 15 und insbesondere die Optik nicht zu beschädigen.

Bei allen Ausführungsformen des Instruments bzw., der Sonde 17 kann die Farbmarkierung 39 oder eine der Farbmarkierungen 39 unmittelbar im Anschluss an das distalen Ende des Instrumentenkörpers 25 angeordnet sein. Alternativ kann die wenigstens eine Farbmarkierung 39 einen Abstand zum distalen Ende 28 aufweisen. Beide Varianten haben ihre eigenen Vorteile.

In den Figuren 3-5, 11 und 12 sind unterschiedliche Ausgestaltungen des Instrumentenkörpers 25 bzw. des Instrumentenschlauchs 26 veranschaulicht. Das Ausführungsbeispiel gemäß Figur 3 entspricht der in Figur 2 veranschaulichten Ausführungsform, bei der das Fluid G bzw. das Plasma in Verlängerung des Endabschnitts 38 ausgestoßen wird. Im Unterschied dazu weist der Instrumentenkörper 25 und beispielsgemäß der Instrumentenschlauch 26 bei den Ausführungsformen gemäß der Figuren 4 und 5 ein Endstück 41 auf, das die Austrittsöffnung 31 aufweist. Über das Endstück 41 kann die Richtung des austretenden Fluid G bzw. des Plasmas variiert werden. Beim Ausführungsbeispiel gemäß Figur 4 tritt das Fluid G bzw. das Plasma im Wesentlichen rechtwinklig zur Erstreckungsrichtung des Endabschnitts 38 in alle Richtungen aus. Demgegenüber ist die Austrittsöffnung 31 am Endstück 41 der Figur 5 so ausgebildet, dass das Inertgas G bzw. das Plasma im Wesentlichen rechtwinklig zur Erstreckungsrichtung des Endabschnitts 38 gezielt seitlich in eine Richtung ausgestoßen wird.

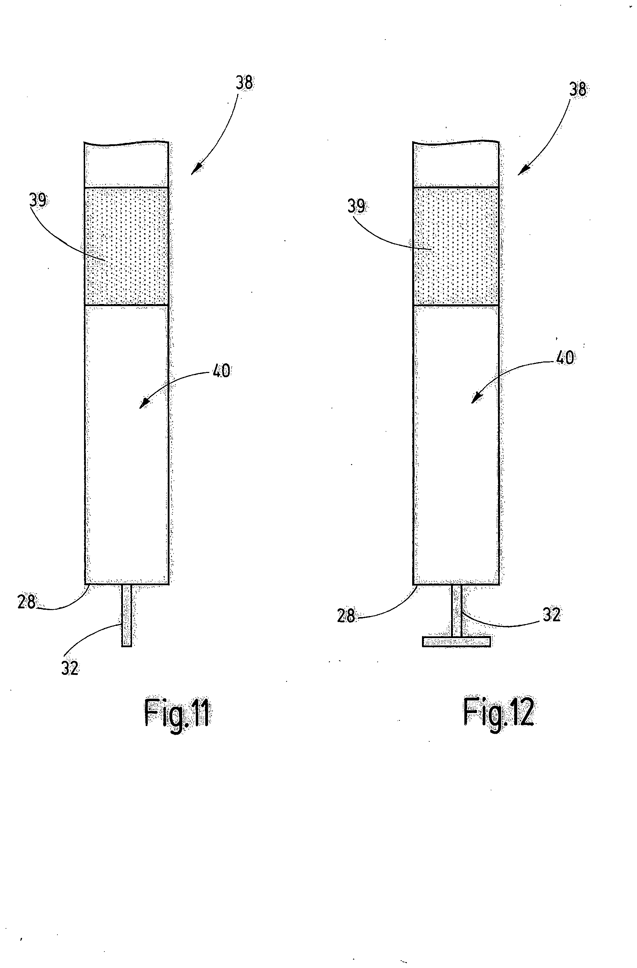

In den Figuren 11 und 12 sind schematisch Ausführungsformen der Sonde dargestellt, bei denen die Elektrode 32 vom distalen Ende 28 des Instrumentenkörpers 25 wegragt. Diese Sonden können ohne Fluidkanal 30 ausgebildet sein. Die Elektrode 32 kann zur thermischen Behandlung direkt in Kontakt mit dem Gewebe 34 gebracht werden oder durch Funkenbildung ohne zusätzliches Medium auf das Gewebe 34 einwirken.

Bei den Ausführungsbeispielen, bei denen ein Endstück 41 vorhanden ist, kann das Endstück 41 eine Farbmarkierung 39 bilden. Hierzu kann es in der definierten braunen Farbe der Farbmarkierung 39 eingefärbt oder ganz oder teilweise beschichtet sein. Das Endstück 41 kann, z.B. aus einem keramischen Material hergestellt sein.

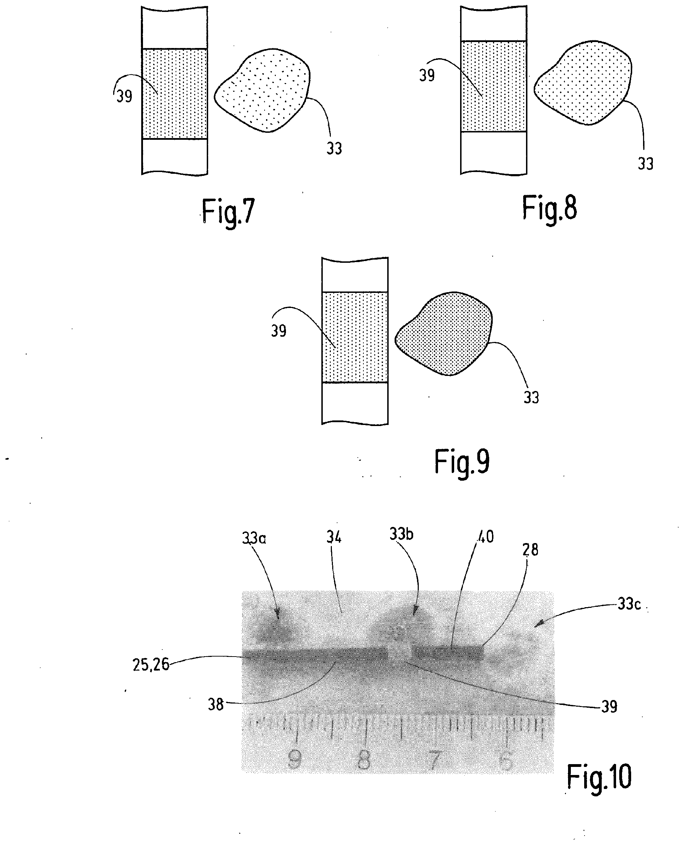

Anhand der Figuren 7-10 ist schematisch die Funktion der wenigstens einen Farbmarkierung 39 erläutert. Durch Positionieren der wenigstens einen Farbmarkierung 39 unmittelbar benachbart zu einem Applikationsbereich 33, innerhalb dem ein Gewebe 34 bereits behandelt wurde, kann ein Farbvergleich durchgeführt werden. Die Helligkeit der Farbe ist in den Figuren 7-9 durch die Dichte der Punkte schematisch dargestellt.

In Figur 7 ist zu erkennen, dass die Farbe der Farbmarkierung 39 dunkler ist als die Farbe des Gewebes 34 im Applikationsbereich 33. Es liegt eine Unterdosierung vor und das Gewebe muss im Applikationsbereich 33 weiter behandelt werden, um die gewünschte Tiefenwirkung zu erreichen.

Demgegenüber ist die Farbe des Gewebes 34 im Applikationsbereich 33 nach Figur 9 dunkler als die Farbe der wenigstens einen Farbmarkierung 39, so dass eine Überdosierung vorliegt. Das Gewebe 34 wurde im Applikationsbereich 33 über die gewünschte Tiefenwirkung hinaus beschädigt bzw. beeinträchtigt. Der Operateur kann dies erkennen und die Folgen abschätzen bzw. gegebenenfalls erforderliche Maßnahmen einleiten.

In Figur 8 ist schematisch die Situation veranschaulicht, bei der die Farbe der Farbmarkierung 39 und die Farbe des behandelten Gewebes im Applikationsbereich 33 in etwa übereinstimmen und der Operateur erkennt, dass die Dosierung zur Erzielung der gewünschten Tiefenwirkung im Gewebe 34 unmittelbar nach der Behandlung (etwa zwei Drittel von der unter Berücksichtigung der Nachwirkung zu erreichten Tiefenwirkung) korrekt ist.

Die in den Figuren 7-9 schematisch veranschaulichten Situationen sind anhand der photographischen Abbildung in Figur 10 nochmals dargestellt. Links im Bild ist ein erster Applikationsbereich 33a zu erkennen, dessen Farbe dunkler ist als die Farbe der Farbmarkierung 39. Es liegt eine Überdosierung vor. Die Farbe in einem zweiten Applikationsbereich 33b entspricht im Wesentlichen der Farbe der wenigstens einen Farbmarkierung 39. In diesem zweiten Applikationsbereich 33b wurde die korrekte Dosierung der eingetragenen Energie erreicht. In einem dritten Applikationsbereich 33c, der eine deutlich hellere Farbe aufweist als die Farbe der Farbmarkierung 39, wurde der Energieeintrag unterdosiert. Durch eine weitere Behandlung dieses dritten Applikationsbereichs 33c kann die gewünschte Dosierung bzw. Tiefenwirkung noch erreicht werden.

Die Erfindung betrifft ein Instrument zur thermischen Behandlung von Gewebe 34, ins besondere zur elektrochirurgischen Behandlung und insbesondere zur Argonplasma-Koagulation. Das Instrument hat einen sich zwischen einem proximalen Ende 27 und einem distalen Ende 28 erstreckenden Instrumentenkörper 25. In einem Endabschnitt 38 angrenzend an das distale Ende 28 weist der Instrumentenkörper 25 wenigstens eine Farbmarkierung 39 in einer definierten Farbe auf. Diese Farbe entspricht der Farbe eines behandelten Gewebes 34, die entsteht, wenn die Dosierung eines Energieeintrags zur Erzielung einer gewünschten Tiefenwirkung im Gewebe 34 korrekt gewählt wurde. Vorzugsweise ist das Instrument als Sonde 17, insbesondere als Endoskopsonde, ausgebildet und hat einen flexibel biegbaren Instrumentenkörper 25, der als Instrumentenschlauch 26 bezeichnet werden kann.

### Bezugszeichenliste:

- 15: Endoskop
- 16: Bedienteil
- 17: Sonde
- 18: Arbeitskanal
- 19: distales Endoskopende
- 20: Lichtquelle
- 21: Objektiv

- 25: Instrumentenkörper
- 26: Instrumentenschlauch
- 27: proximales Ende des Instrumentenschlauchs
- 28: distales Ende des Instrumentenschlauchs
- 29: Versorgungs- und Bedieneinheit
- 30: Fluidkanal
- 31: Austrittsöffnung
- 32: Elektrode
- 33: Applikationsbereich
- 33a: erster Applikationsbereich
- 33b: zweiter Applikationsbereich
- 33c: dritter Applikationsbereich
- 34: Gewebe
- 35: Plasma

- 38: Endabschnitt
- 39: Farbmarkierung
- 40: Mantelfläche
- 41: Endstück

- d: Mindestabstand
- E: Richtung des elektrischen Feldes
- G: Fluid

## Patentansprüche

1. Instrument zur thermischen Behandlung von Gewebe (34) durch Eintragen von Energie auf das zu behandelnde Gewebe (34) in einem Applikationsbereich (33),
mit einem Instrumentenkörper (25), der sich von einem proximales Ende (27) zu einem distalen Ende (28) erstreckt,
mit einer Elektrode (32), zum Anlegen einer elektrischen Spannung eingerichtet ist,
**dadurch gekennzeichnet, dass** im Bereich des distalen Endes (28) am Instrumentenkörper (25) wenigstens eine Farbmarkierung (39) von außen sichtbar angeordnet ist, deren Farbe derart gewählt ist, dass sie der sichtbaren Farbe des behandelten Gewebes (34) im Applikationsbereich (33) mit der gewünschten Dosierung und/oder Tiefenwirkung entspricht, wobei die Dosierung den Energieeintrag in das zu behandelnden Gewebe (34) beschreibt.

2. Instrument nach Anspruch 1,
**dadurch gekennzeichnet, dass** die Farbe der wenigstens einen Farbmarkierung (39) braun oder braunbeige ist.

3. Instrument nach Anspruch 1 oder 2,
**dadurch gekennzeichnet, dass** die Farbe der wenigstens einen Farbmarkierung (39) eine RAL-Farbe ist.

4. Instrument nach einem der vorhergehenden Ansprüche, **dadurch gekennzeichnet, dass** die Farbmarkierung (39) eine Länge in Erstreckungsrichtung des Instrumentenkörpers (25) von mindestens 2 mm oder 3 mm aufweist.

5. Instrument nach einem der vorhergehenden Ansprüche, **dadurch gekennzeichnet, dass** die wenigstens eine Farbmarkierung (39) an einer Mantelfläche (40) des Instrumentenkörpers (25) vorhanden ist.

6. Instrument nach Anspruch 5,
**dadurch gekennzeichnet, dass** zumindest ein Abschnitt der wenigstens einen Farbmarkierung (39) einen definierten Mindestabstand (d) zum distalen Ende (28) angibt.

7. Instrument nach Anspruch 6,
**dadurch gekennzeichnet, dass** der vom distalen Ende (28) am weitesten entfernte Rand der wenigstens einen Farbmarkierung (39) den Mindestabstand (d) zum distalen Ende (28) angibt.

8. Instrument nach einem der Ansprüche 5 bis 7,
**dadurch gekennzeichnet, dass** sich die wenigstens eine Farbmarkierung (39) in einer Umfangsrichtung um mindestens 50% des Umfangs des Instrumentenkörpers (25) erstreckt.

9. Instrument nach Anspruch 8,
**dadurch gekennzeichnet, dass** sich die wenigstens eine Farbmarkierung (39) vollständig um den Umfang des Instrumentenkörpers (28) erstreckt.

10. Instrument nach einem der vorhergehenden Ansprüche, **dadurch gekennzeichnet, dass** die wenigstens eine Farbmarkierung (39) einen Abstand zur Austrittsöffnung (31) aufweist.

11. Instrument nach einem der vorhergehenden Ansprüche, **dadurch gekennzeichnet, dass** die wenigstens eine Farbmarkierung (39) an einem Endstück (41) des Instrumentenkörpers (25) vorhanden ist, das die Austrittsöffnung (31) aufweist.

12. Instrument nach einem der vorhergehenden Ansprüche, **dadurch gekennzeichnet, dass** der Instrumentenkörper (25) als Instrumentenschlauch (26) ausgebildet ist.

13. Instrument nach Anspruch 12,
**dadurch gekennzeichnet, dass** der Instrumentenschlauch (26) dazu eingerichtet ist, durch einen Arbeitskanal (18) eines Endoskops (15) geführt zu werden.

14. Instrument nach einem der vorhergehenden Ansprüche, **dadurch gekennzeichnet, dass** das Instrument zur Plasmabehandlung von Gewebe (34) eingerichtet ist.

15. Instrument nach einem der vorhergehenden Ansprüche, **dadurch gekennzeichnet, dass** in dem Instrumentenkörper (25) ein Fluidkanal (30) vorhanden ist, der fluidisch mit einer Austrittsöffnung (31) im Bereich des distalen Endes (28) verbunden ist, und wobei die Elektrode (32) innerhalb des Instrumentenkörpers (25) benachbart zur Austrittsöffnung (31) angeordnet ist.

## Claims

1. Instrument for thermal treatment of tissue (34) by introducing energy into tissue (34) to be treated in the application area (33),
having an instrument body (25) that extends from a proximal end (27) to a distal end (28),
having an electrode (32) configured for application of an electric voltage,
**characterized in that** in the area of the distal end (28) at least one color mark (39) is arranged on the instrument body (25) visible from the outside, wherein the color of the color mark (39) is selected such that it corresponds to the visible color of the treated tissue (34) in the application area (33) treated with the desired dosage and/or depth effect, wherein the dosage describes the introduction of energy into the tissue (34) to be treated.

2. Instrument according to claim 1, **characterized in that** the color in the at least one color mark (39) is brown or brown-beige.

3. Instrument according to claim 1 or 2, **characterized in that** the color of the at least one color mark (39) is a RAL-color.

4. Instrument according to any of the preceding claims, **characterized in that** the color mark (39) has a length in extension direction of the instrument body (25) of at least 2 mm or 3 mm.

5. Instrument according to any of the preceding claims, **characterized in that** the at least one color mark (39) is provided at a shell surface (40) of the instrument body (25) .

6. Instrument according to claim 5, **characterized in that** at least a section of the at least one color mark (39) indicates a defined minimum distance (d) from the distal end (28).

7. Instrument according to claim 6, **characterized in that** the edge of the at least one color mark (39) that is furthest away from the distal end (28) indicates the minimum distance (d) from the distal end (28).

8. Instrument according to any of the claims 5-7, **characterized in that** the at least one color mark (39) extends in a circumferential direction about at least 50% of the circumference of the instrument body (25).

9. Instrument according to claim 8, **characterized in that** the at least one color mark (39) extends completely around the circumference of the instrument body (25).

10. Instrument according to any of the preceding claims, **characterized in that** the at least one color mark (39) has a distance to an exit opening (31).

11. Instrument according to any of the preceding claims, **characterized in that** the at least one color mark (39) is provided on an end piece (41) of the instrument body (25) comprising an exit opening (31).

12. Instrument according to any of the preceding claims, **characterized in that** the instrument body (25) is configured as instrument hose (26).

13. Instrument according to claim 12, **characterized in that** the instrument hose (26) is configured to be guided through an operation channel (18) of the endoscope (15).

14. Instrument according to any of the preceding claims, **characterized in that** the instrument is configured for plasma treatment of tissue (34).

15. Instrument according to any of the preceding claims, **characterized in that** a fluid channel (30) is provided in the instrument body (25) that is fluidically connected with an exit opening (31) in the area of the distal end (28), whereby the electrode (32) is arranged inside the instrument body (25) adjacent to the exit opening (31).

## Revendications

1. Instrument de traitement thermique de tissus (34), par apport d'énergie sur le tissu (34) à traiter, dans une zone d'application (33),
comprenant un corps d'instrument (25) qui s'étend depuis une extrémité proximale (27) jusqu'à une extrémité distale (28),
comprenant une électrode (32) destinée à appliquer une tension électrique,
**caractérisé en ce qu'**il est prévu sur le corps d'instrument (25), dans la région de l'extrémité distale (28), au moins un repère de couleur (39) qui est visible de l'extérieur et dont la couleur est choisie de manière à ce qu'elle corresponde à la couleur visible du tissu (34) traité, dans la zone d'application (33) avec le dosage et/ou l'action en profondeur souhaités, le dosage décrivant l'apport d'énergie dans le tissu (34) à traiter.

2. Instrument selon la revendication 1, **caractérisé en ce que** la couleur du repère de couleur (39), au nombre d'au moins un, est le brun ou le beige brun.

3. Instrument selon la revendication 1 ou 2, **caractérisé en ce que** la couleur du repère de couleur (39), au nombre d'au moins un, est une couleur RAL.

4. Instrument selon une des revendications précédentes, **caractérisé en ce que** le repère de couleur (39) présente une longueur dans le sens de l'étendue du corps d'instrument (25) qui est au moins de 2 mm ou de 3 mm.

5. Instrument selon une des revendications précédentes, **caractérisé en ce que** le repère de couleur (39), au nombre d'au moins un, est présent sur une surface enveloppe (40) du corps d'instrument (25).

6. Instrument selon la revendication 5, **caractérisé en ce qu'**au moins une partie du repère de couleur (39), au nombre d'au moins un, indique une distance minimale (d) définie par rapport à l'extrémité distale (28).

7. Instrument selon la revendication 6, **caractérisé en ce que** le bord du repère de couleur (39), au nombre d'au moins un, qui est le plus éloigne de l'extrémité distale (28), indique la distance minimale (d) par rapport à l'extrémité distale (28).

8. Instrument selon une des revendications 5 à 7, **caractérisé en ce que** le repère de couleur (39), au nombre d'au moins un, s'étend dans une direction périphérique autour d'au moins 50 % du pourtour du corps d'instrument (25).

9. Instrument selon la revendication 8, **caractérisé en ce que** le repère de couleur (39), au nombre d'au moins un, s'étend entièrement autour du pourtour du corps d'instrument (28).

10. Instrument selon une des revendications précédentes, **caractérisé en ce que** le repère de couleur (39), au nombre d'au moins un, présente une distance par rapport à l'orifice de sortie (31).

11. Instrument selon une des revendications précédentes, **caractérisé en ce que** le repère de couleur (39), au nombre d'au moins un, est présent sur une pièce d'extrémité (41) du corps d'instrument (25), qui présente l'orifice de sortie (31).

12. Instrument selon une des revendications précédentes, **caractérisé en ce que** le corps d'instrument (25) est réalisé sous forme de tuyau d'instrument (26).

13. Instrument selon la revendication 12, **caractérisé en ce que** le tuyau d'instrument (26) est agencé pour être guidé dans un canal de travail (18) d'un endoscope (15).

14. Instrument selon une des revendications précédentes, **caractérisé en ce que** l'instrument est agencé pour le traitement au plasma de tissus (34).

15. Instrument selon une des revendications précédentes, **caractérisé en ce que** dans le corps d'instrument (25), il est prévu un canal de fluide (30) qui est relié sur le plan fluidique à un orifice de sortie (31) dans la région de l'extrémité distale (28), et l'électrode (32) étant disposée à l'intérieur du corps d'instrument (25), dans le voisinage de l'orifice de sortie (31).
